## Europäisches Patentamt

## European Patent Office

## Office européen des brevets

(11) Publication number: **0 166 264**
**B1**

## EUROPEAN PATENT SPECIFICATION

(45) Date of publication of patent specification: **12.10.88**

(51) Int. Cl.⁴: **C 07 C 118/00, C 08 G 18/70, C 08 G 18/48**

(21) Application number: **85106698.5**

(22) Date of filing: **30.05.85**

(54) **Process for preparing aqueous emulsions of polyisocyanates.**

(30) Priority: **30.05.84 IT 2116084**

(43) Date of publication of application:
**02.01.86 Bulletin 86/01**

(45) Publication of the grant of the patent:
**12.10.88 Bulletin 88/41**

(84) Designated Contracting States:
**BE DE FR GB NL SE**

(56) References cited:
**FR-A-2 378 810**

(73) Proprietor: **A.TE.CA. S.r.l.**
**31, Foro Buonaparte**
**Milan (IT)**

(72) Inventor: **Pallozzi, Franco**
**18, via Duca degli Abruzzi**
**Vimercate (Milano) (IT)**
Inventor: **Manucra, Fortunato**
**59, via Vittorio Veneto**
**Castellanza (Varese) (IT)**
Inventor: **Turco, Pietro**
**13, via Venezia**
**Busto Arsizio (Varese) (IT)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

The file contains technical information submitted after the application was filed and not included in this specification

Courier Press, Leamington Spa, England.

## Description

This invention relates to a process for preparing aqueous emulsions of polyisocyanates.

It is known in the art to prepare polyisocyanate emulsions by using surfactants, generally anionic surfactants, having a molecular weight not higher than 1000—1500, which contain hydrophobic groups with a polyisocyanate structure (for example urethane bonds, according to GB—A—1,444,933) and hydrophilic groups (for example a polyoxyethylene chain). It is also known to use, for this purpose, anionic surfactants based on hexamethylenediamine (as hydrophobic moiety) and polyoxymethylene (as hydrophilic moiety) optionally modified with polyoxypropylene, according to what is described, for example, in GB—A—2,039,780.

FR—A—237 8810 discloses a process for the preparation of aqueous emulsions of polyisocyanates wherein the polyisocyanate is dispersed in water in the presence of e.g. a polyglycol having a molecular weight between 4000 and 20 000. As can be seen from the examples of said document, the polyglycol is efficient as emulsifier when the polyisocyanate is added to the water quickly and with a very high stirring speed.

The present invention relates to a process for preparing aqueous emulsions of polyisocyanates, which differs from the methods of the prior art reported hereinabove, and which permits to obtain generally more stable emulsions.

The process of the present invention consists in gradually adding the polyisocyanate under stirring to an aqueous solution containing 1 to 5% by weight of a polyoxyethylene glycol having an average molecular weight ranging from 350,000 to 600,000, but preferably from 400,000 to 500,000.

The addition of the polyisocyanate to the aqueous solution of the polyoxyethylene glycol is carried out with such a stirring speed and addition rate as to permit a practically immediate mixing of polyisocyanate and water.

For example, if a Cowles stirrer is utilized, the conditions for an immediate mixing occur at an impeller speed of 1800—2200 rpm and at a polyisocyanate feeding rate ranging approximately from about 50 to 80 g/minute.

The temperature of the polyoxyethylene glycol solution is preferably not higher than 30°C, and generally ranges from 10° to 30°C.

The polyisocyanate utilizable for the purposes of the present invention may be either hexamethylene diisocyanate, or an aromatic polyisocyanate, such as 2,4- and 2,6 - toluene diisocyanate, 4,4' - diphenylmethane diisocyanate and diphenyl - 4,4' - diisocyanate. Polymethylene - polyphenylisocyanate having a mean functionality of 2.7—2.8, obtained by phosgenation of the polyamines derived from the condensation of aniline with formaldehyde has proved to be a particularly suitable polyisocyanate. Such product is commercially available under various trade-names, such as "Tedimon®" 31 (produced by Montepolimeri), "PaPi®" (produced by Up John) and "Hondur® MR" (produced by Mobay).

The following examples are given to illustrate the present invention.

Examples 1—7

Polyoxyethylene glycol having an average molecular weight of 400,000 was added under stirring to water, optionally heated to 60°C, until dissolution, in amounts of 0.5%, 1.5%, 2% and 3% by weight, respectively.

To these solutions maintained at 20—25°C there was added polymethylene-polyphenyisocyanate (Tedimon® 31) under stirring, supplied by a Cowles-type stirrer, at a speed higher than 2000 rpm. The addition of polyisocyanate was effected at a rate of 50—80 g/minute, with polyisocyanate/water weight ratios of 30, 40 and 50, respectively.

The characteristics of the emulsions so prepared and the ageing time as a function of the concentration of polyoxyethyleneglycol and of polyisocyanate are indicated in Table I and in Table II.

The percentage of —NCO groups, which remains at acceptable levels even after a 24-hour ageing, is indicative of the applicability of the emulsions. From the data of the tables it is apparent that the concentration of the polyisocyanate in the emulsion does not substantially affect the stability and applicability of the emulsion.

Example 8

An emulsion containing 40% of active substance, prepared according to the above described method, was utilized to impregnate an amount of wooden shavings suitable for the preparation of six three-layer shaving panels; the concentration of polyisocyanate based on the dry shavings was 6% in the inner layer and 8% in the outer layers.

The panels, prepared according to the usual conditions employed also for the ones based on ureic products (15"/mm at a press temperature around 180°C), were characterized according to DIN 68763 V100 and gave the following results:

0 166 264

|  | Panels without paraffin | Panels with paraffin | Standard DIN 68763 values type V100 |
|---|---|---|---|
| Flexural strength kg/cm² | 185 | 190 | ≥180 |
| Tensile strength kg/cm² | 2.2 | 2.7 | ≥1.5 |
| Swelling % | 9.8 | 7.5 | ≤12 |
| Density g/cm³ | 0.63 | 0.62 | — |
| Thickness mm | 16 | 16 | 13—20 |

TABLE 1
Stability of polymethylene-polyphenylisocyanate emulsions (at 40% in water)

| Emulsion characteristics / Ageing time (hours) | 3% Polyoxyethylene glycol | | 2% Polyoxyethylene glycol | | 1,5% Polyoxyethylene glycol | | 0,5% Polyoxyethylene glycol | |
|---|---|---|---|---|---|---|---|---|
| | %NCO | Visc. at 25°C cF4 (sec.) | %NCO | Visc. at 25°C cF4 (sec.) | %NCO | Visc. at 25°C cF4 (sec.) | %NCO | Visc. at 25°C cF4 (sec.) |
| 0 | 100 | 144 | 100 | 40 | 100 | 24 | 100 | |
| 1 | | | | | | | | |
| 2 | 100 | | 96 | | 95 | | | |
| 3 | | | | 34 | | | | sharp separation |
| 4 | | 132 | 94 | | | 21 | | |
| 5 | 93 | | | | 93 | | | |
| 6 | | | | | | | | |
| 7 | 93 | | 93 | 32 | 93 | | | |
| 8 | | 100 | 93 | | 93 | 18 | | |
| 9 | 82 | | | 31 | | | | |
| 10 | | 95 | | | | 19 | | |
| 11 | | | | | | | | |
| 12 | | | | 28 | | | | |
| 13 | | 95 | | | | 18 | | |
| 14 | 75 | | 81 | 28 | 82 | 18 | | |

TABLE 2
Stability of polymethylene-polyphenylisocyanate emulsions at the same concentration of
2% of polyoxyethylene glycol on polyisocyanate

| Emulsion characteristics / Ageing time (hours) | Polyisocyanate/ $H_2O=50$ | | Polyisocyanate/ $H_2O=40$ | | Polyisocyanate/ $H_2O=30$ | |
|---|---|---|---|---|---|---|
| | %NCO | Visc. at 25°C cF4 (sec.) | %NCO | Visc. at 25°C cF4 (sec.) | %NCO | Visc. at 25°C cF4 (sec.) |
| 0 | 100 | 48 | 100 | 40 | 100 | 23 |
| 1 | | | | | | |
| 2 | 98,7 | | 96,2 | | 99,4 | |
| 3 | | | | 34 | | 23 |
| 4 | 97 | 41 | 93,7 | | 96,4 | |
| 5 | | | | | | |
| 6 | | | 93,1 | 32 | 90 | |
| 7 | 96,3 | | | | | 22 |
| 8 | | 32 | 93 | 31 | 88 | |
| 9 | 96 | | | | | 22 |
| 10 | 94,5 | 32 | | | 87 | 21,5 |
| 11 | | 32 | | 28 | | |
| 12 | | | | | | |
| 13 | | | 81 | | | |
| 14 | 89,6 | 32 | 81 | 28 | 85 | 20 |

**Claims**

1. A process for preparing a stable aqueous emulsion of a polyisocyanate which consists in adding the polyisocyante to an aqueous solution containing 1 to 5% by weight of a polyoxyethylene glycol, characterized in that the polyoxyethylene glycol has an average molecular weight of from 350,000 to 600,000 and is added to the aqueous solution with such a stirring speed and addition rate as to permit a practically immediate mixing of polyisocyanate and water.

2. The process according to Claim 1, in which the polyoxyethylene glycol solution has a temperature not higher than 30°C.

3. The process according to Claims 1 and 2, in which the polyisocyanate is polymethylene-polyphenylisocyanate having a mean functionality of 2.7—2.8.

4. The process according to Claims 1 to 3, in which the polyoxyethylene glycol has an average molecular weight ranging from 400,000 to 500,000.

**Patentansprüche**

1. Verfahren zur Herstellung einer stabilen wässrigen Emulsion eines Polyisocyanates, das besteht aus: der Zugabe des Polyisocyanates zu einer wässrigen, 1 bis 5 Gew.-% eines Polyoxyethylenglykols enthaltenden Lösung, dadurch gekennzeichnet, daß das Polyoxyethylenglykol ein durchschnittliches Molekulargewicht von 350 000 bis 600 000 hat und zur wässrigen Lösung mit solcher Rührgeschwindigkeit und Zugabegeschwindigkeit zugegeben wird, daß ein praktisch sofortiges Mischen von Polyisocyanat und Wasser ermöglicht wird.

2. Verfahren nach Anspruch 1, in welchen die Polyoxyethylenglykollösung eine Temperatur nicht höher als 30°C hat.

3. Verfahren nach Anspruch 1 und 2, in welchem das Polyisocyanat Polymethylen-Polyphenylisocyanat mit einer mittleren Funktionalität von 2,7 bis 2,8 ist.

4. Verfahren nach Anspruch 1 bis 3, in welchem das Polyoxyethylenglykol ein durchschnittliches Molekulargewicht zwischen 400 000 bis 500 000 hat.

**Revendications**

1. Un procédé de préparation d'une émulsion aqueuse stable d'un polyisocyanate qui consiste à ajouter le polyisocyanate à une solution aqueuse contenant de 1 à 5% en poids d'un polyoxyéthylèneglycol, caractérisé en ce que le poids moléculaire moyen du polyoxyéthylèneglycol est compris entre 350 000 et 600 000 et en ce qu'on l'ajoute à la solution aqueuse à une vitesse d'agitation et à une vitesse d'addition telles qu'elle permette un mélange pratiquement immédiat du polyisocyanate et de l'eau.

2. Le procédé selon la revendication 1, dans lequel la solution de polyoxyéthylèneglycol est à une température ne dépassant pas 30°C.

3. Le procédé selon les revendications 1 et 2, dans lequel le polyisocyanate est le polyméthylène-polyphénylisocyanate présentant une fonctionnalité moyenne comprise entre 2,7 et 2,8.

4. Le procédé selon les revendications 1 à 3, dans lequel le polyoxyéthylèneglycol présente un poids moléculaire moyen compris entre 400 000 et 500 000.